# EUROPÄISCHE PATENTANMELDUNG

(11) **EP 2 628 501 A1**
(43) Veröffentlichungstag der Anmeldung: **21.08.2013**
(21) Anmeldenummer: 12196254.2
(22) Anmeldetag: 10.12.2012
(51) Int. Cl.: A61N 1/05

(54) **Elektrodenvorrichtung, insbesondere zur kardiovaskulären Anwendung**

(30) Priorität: 16.02.2012 US 201261599429 P
(71) Anmelder: BIOTRONIK SE & Co. KG, 12359 Berlin (DE)
(72) Erfinder: Bartels, Klaus, 10115 Berlin (DE); Günther, Thomas, 14552 Michendorf (DE); Steglich, Carsten, 12587 Berlin (DE)
(74) Vertreter: Lindner-Vogt, Karin L.

(57) **Zusammenfassung**

Eine Elektrodenvorrichtung, insbesondere zur kardiovaskulären Anwendung, umfasst einen lang gestreckten Elektrodenkörper (1) aus einem isolierenden Material, mehrere Elektroden (4, 5) zur Erfassung elektrokardiologischer Signale und/oder zur Abgabe elektrokardiologischer Reizsignale, und für die elektrische Anbindung der Elektroden (4, 5) dienende Zuleitungen (2, 2S, 2D), insbesondere nicht-dehnbare Seile oder Litzen, welche Zuleitungen (2, 2S 2D) jeweils im Elektrodenkörper (1) vorzugsweise in zugeordneten Lumen (3, 13) geführt sind. Ferner ist ein in eine Trennstelle (T) im Elektrodenkörper (1) eingefügter Ausgleichs-Schlauchabschnitt (7) mit maximal dem Elektrodenkörper (1) entsprechenden Außendurchmesser (DA) vorgesehen, wobei in den Ausgleichs-Schlauchabschnitt (7) schraubenlinienförmige Aufnahmen (8) für jeweils eine Zuleitung (2) eingebracht sind und der Ausgleichs-Schlauchabschnitt (7) an seinen Fügeseiten (9) zum Elektrodenkörper (1) hermetisch dicht mit diesem verbunden ist.

## Beschreibung

Die Erfindung betrifft eine Elektrodenvorrichtung insbesondere zur kardiovaskulären Anwendung mit den im Oberbegriff des Anspruches 1 angegebenen Merkmalen.

Derartige Elektrodenvorrichtungen, wie sie beispielsweise aus der US 7,238,883 B2, US 2007/0225784 A1 oder US 7,395,116 B2 bekannt sind, weisen bekanntermaßen einen langgestreckten Elektrodenkörper aus einem isolierenden Material, eine oder mehrere Elektroden zur Erfassung kardiologischer Signale und/oder zur Abgabe elektrokardiologischer Reizsignale sowie für die elektrische Anbindung der Elektroden dienende Zuleitungen auf. Letztere sind jeweils in entsprechenden Lumen im Elektrodenkörper geführt.

Solche Elektrodenvorrichtungen, wie sie beispielsweise als Schrittmacher-, Defibrillator- und andere Multielektrodensysteme Anwendung finden, werden für diagnostische und therapeutische Zwecke genutzt. Als Zuleitungen werden dabei immer häufiger nicht-dehnbare elektrische Leiter, wie beispielsweise Seile und Litzen eingesetzt.

Infolge der exzentrischen Anordnung mehrerer Leiter dieses nicht-dehnbaren Typs sowohl in Multilumen-Konstruktionen als auch in lumenlosen Anordnungen ist eine Relativbewegung zwischen diesen Leitern und der Elektrodenisolation bei der Multilumenanordnung bzw. zwischen den isolierten Zuleitungen untereinander bei einem lumenlosen Konstrukt unvermeidlich. Dies führt naturgemäß bei ein oder mehreren Seile, die in ihren Führungslumen des isolierenden Elektrodenkörpers ein geringes Spiel zeigen, die ferner in der Regel bezogen auf ihren Querschnitt radial außen bzw. asymmetrisch um ein einen zentralen Wendelleiter führendes Lumen angeordnet sind und damit nicht in der neutralen Phase des Elektrodenkörpers liegen, je nach Spannungszustand zu Reibungen zwischen den beteiligten Bauteilen, die insbesondere bei großen Biegebewegungen innerhalb eines Elektrodenkörpers im Vergleich mit einem konventionellen koaxialen Aufbau, bestehend aus jeweils dehnbaren Innen- und Außenleitern in Form von Wendeln, erheblich sein können.

Elektrodenvorrichtungen mit mehreren Einzelelektroden, wie sie zur Stimulation und Wahrnehmung von Herzaktionspotentialen, zur Defibrillation oder zum Anschluss von Sensoren bzw. Aktuatoren eingesetzt werden, bestehen zur Optimierung des Fertigungsablaufes ferner häufig aus vorgefertigten Baugruppen, die im Verlauf der Fertigung zusammengefügt werden müssen. Es ist in diesem Zusammenhang bei marktüblichen Elektrodenvorrichtungen bekannt, solche Verbindungen mittels Verklebung einer als Verbindungsmuffe dienenden Übergabehülse herzustellen, die als eine Art "Bandage" um die Fügestelle zwischen den vorgefertigten Teilkomponenten wirkt. So kann beispielsweise eine Schockelektroden-Baugruppe einer Defibrillator-Elektrodenvorrichtung mit dem eigentlichen langgestreckten Elektrodenkörper auf die vorstehende Art und Weise konfektioniert werden. Die Übergänge mit Übergabehülsen können dabei Störstellen in Form von Durchmessersprüngen in der ansonsten isodiametrischen Gestaltung des Elektrodenkörpers darstellen, die nach einer Implantation der Elektrodenvorrichtung Ursache von verstärktem Gewebebewuchs sein können, der die Funktion des Systems beeinträchtigen kann.

Aus dem Stand der Technik ist es durch offenkundige Vorbenutzung bekannt, das Problem der nicht-dehnbaren Seile und Litze als Leiter durch eine Seilverdrillung zu lösen. Diese Maßnahme ist jedoch nur direkt bei der Anbindung des Seiles bzw. der Litze an einen Stecker bekannt und dient dort ausschließlich der Erhöhung der Biegelast-Wechselfähigkeit am Übergang zwischen Leiterseil und Stecker.

Eine weitere bekannte Lösung ist die Übergabe des Leiterseiles in eine dehnbare Wendel. Problematisch hierbei ist die Tatsache, dass durch diesen Übergang zwischen einem Seil und einer Drahtwendel im Hochspannungspfad einer Elektrodenvorrichtung die elektrischen Eigenschaften der Elektrodenvorrichtung signifikant verschlechtert werden. Insoweit ist dieser Stand der Technik nicht praktikabel.

Ausgehend von den geschilderten Problemen des Stand der Technik liegt der Erfindung die Aufgabe zugrunde, eine Elektrodenvorrichtung so weiterzubilden, dass Bewegungen der Zuleitungen, insbesondere wenn diese als nicht-dehnbare Seile oder Litzen ausgebildet sind, relativ zum isolierenden Elektrodenkörper minimiert und bei der Umsetzung Verbindungsmuffen oder Übergabehülsen bei entsprechender Auslegung der Elektrodenvorrichtung vermieden werden.

Diese Aufgabe wird durch die im Kennzeichnungsteil des Anspruches 1 angegebenen Merkmale gelöst. Demnach ist ein in eine Trennstelle im Elektrodenkörper eingefügter Ausgleichs-Schlauchabschnitt mit maximal dem Elektrodenkörper entsprechenden Außendurchmesser vorgesehen. In diesen Ausgleichs-Schlauchabschnitt sind schraubenlinienförmige Aufnahmen für jeweils eine Zuleitung eingebracht. Ferner ist der Ausgleichs-Schlauchabschnitt mit seinen Fügeseiten zum Elektrodenkörper hin hermetisch dicht mit diesem verbunden.

Durch das Legen der Zuleitungen in eine schraubenlinienförmige Konfiguration im Bereich des Ausgleichs-Schlauchabschnittes kann der Elektrodenkörper im Bereich dieses Schlauchabschnittes problemlos Biegeeinflüssen unterzogen werden, da Zugbelastungen auf den Leitungsabschnitt, der an der Außenseite der Verbiegung liegt, durch die Stauchung dieser Zuleitung an der Innenseite der Biegung innerhalb des Ausgleichs-Schlauchabschnittes kompensiert werden. Insoweit ist der Elektrodenkörper im Bereich dieses Ausgleichs-Schlauchabschnittes also besonders biegsam, ohne dass nennenswerte interne Reibungskräfte erzeugt werden. Mit Vorteil sind solche Elektrodenvorrichtungen aufgrund der erhöhten Flexibilität im Bereich der Ausgleichs-Schlauchabschnitte physiologisch verträglicher. Bezüglich der Produktsicherheit werden Elektrodenvorrichtungen mit Mehrlumenaufbauten zuverlässiger, da sie unempfindlicher gegen Biegelastwechsel werden. Dies wird auch durch die verringerte Reibung zwischen den Leitern im benachbarten Lumen bei einem Multilumenaufbau bzw. zwischen benachbarten isolierten Leitern bei einem lumenlosen Konstrukt begünstigt.

Durch die Anbindung der Ausgleichs-Schlauchabschnitte an den benachbarten Elektrodenkörper über eine hermetisch dichte Verbindung an seinen Fügeseiten zum Elektrodenköper hin werden ferner Übergabehülsen und die entsprechenden Arbeitsschritte für deren Anbringung eingespart. Durch die hülsenlose Konstruktion werden auch Durchmessersprünge vermieden und ein isodiametrischer Aufbau der Elektrodenvorrichtung gewährleistet.

Gemäß einer bevorzugten Ausführungsform der Elektrodenvorrichtung können die Aufnahme für die Zuleitungen im Ausgleichs-Schlauchabschnitt als schraubenlinienförmige Lumen oder auf der Außenseite offene, schraubenlinienförmige Nuten ausgebildet sein.

Im letztgenannten Fall ist der Ausgleichs-Schlauchabschnitt vorzugsweise mit seinen die Zuleitungen aufnehmenden Nuten außenseitig mit einer Abdeckhülse umschlossen. Im Gegensatz zum Stand der Technik ist dies allerdings hinsichtlich eines isodiametrischen Aufbaus der Elektrodenvorrichtung unproblematisch, da dann der Ausgleichs-Schlauchabschnitt einen um die Wandstärke der Abdeckhülse verringerten Radius aufweist.

Eine besonders bevorzugte Ausführungsform der Erfindung ergibt sich in ihrer Anwendung bei einer Elektrodenvorrichtung, die mindestens eine Schockwendel auf dem Elektrodenkörper aufweist. Letztere ist in der Regel aus einem leitfähigen, schraubenlinienförmig gewickelten Wendeldraht gebildet. Besonders bevorzugt ist dann die Anordnung des Ausgleichs-Schlauchabschnittes zumindest teilweise unter dieser Schockwendel, wobei mindestens eine Fügeseite des Ausgleichs-Schlauchabschnittes zum Elektrodenkörper hin unter der Schockwendel liegt. Der Elektrodenkörper greift dann mit einem im Durchmesser reduzierten Absatz in die Schockwendel bis zur Fügeseite des Ausgleichs-Schlauchabschnittes ein.

In vorteilhafter Weise ist damit die Trennstelle zwischen Elektrodenkörper und Ausgleichs-Schlauchabschnitt unter der Schockwendel platziert, was der Ausbildung einer ideal homogenen, isodiametrischen Elektrodenvorrichtung zuträglich ist. Die im Durchmesser reduzierten Absätze des Elektrodenkörpers können dabei spanend durch Fräsen oder auch durch Schleifen oder Laserabtrag hergestellt werden.

Besonders geschützt ist die Übergabestelle zwischen Ausgleichs-Schlauchabschnitt und Elektrodenkörper, wenn die Zwischenräume zwischen dem Schlauchabschnitt und der Schockwendel mit einem Vergussmittel verfüllt werden.

Sofern die Elektrodenvorrichtung eine zentral geführte, dehnbare Zuleitung und insbesondere Wendelleitung umfasst, ist es als weitere bevorzugte Ausführungsform vorgesehen, dass der Ausgleichs-Schlauchabschnitt ein zentrales, geradlinig durchgehendes Lumen für diese dehnbare Zuleitung aufweist. Damit kann der Ausgleichs-Schlauchabschnitt breitbandig für eine Vielzahl unterschiedlicher Zuleitungskonfigurationen eingesetzt werden.

Weitere bevorzugte Ausführungsformen beziehen sich auf die Materialauswahl und entsprechende Bereitstellung des Ausgleichs-Schlauchabschnitt, der aus Silikongummi bestehen kann. Die Herstellung kann durch Extrusion oder Spritzgießen aus Flüssig-Silikongummi erfolgen.

Bei Verwendung einer Abdeckhülse für die Trennstelle ist es materialtechnisch von Vorteil, den Ausgleichs-Schlauchabschnitt aus Silikongummi oder Silikon-PolyurethanCopolymer und die Abdeckhülse aus letztgenanntem Material oder Polyurethan herzustellen. Letztgenannte Materialien weisen gute Abriebeigenschaften auf, die insbesondere für die äußere Abdeckhülse relevant sind.

Schließlich hat sich eine Ganghöhe der schraubenlinienförmigen Aufnahmen im Ausgleichs-Schlauchabschnitt als vorteilhaft hinsichtlich eines Ausgleichs von Biegebelastungen und entsprechender Reduzierung der inneren Reibung erwiesen, die dem 3- bis 5-fachen des Außendurchmessers des Ausgleichs-Schlauchabschnittes entspricht. Damit ist auch das Maß der Verlängerung der Zuleitungen im Bereich des Ausgleichsabschnittes auf ein akzeptables Maß beschränkt.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: eine ausschnittsweise, teilweise weggebrochene Seitenansicht einer Elektrodenvorrichtung in einer ersten Ausführungsform,
- Fig. 2: eine Ansicht der Elektrodenvorrichtung gemäß Fig. 1 in einer Explosionsdarstellung,
- Fig. 3: eine Seitenansicht einer Elektrodenvorrichtung in einer zweiten Ausführungsform, und
- Fig. 4: eine Ansicht der Elektrodenvorrichtung gemäß Fig. 3 in einer Explosionsdarstellung.

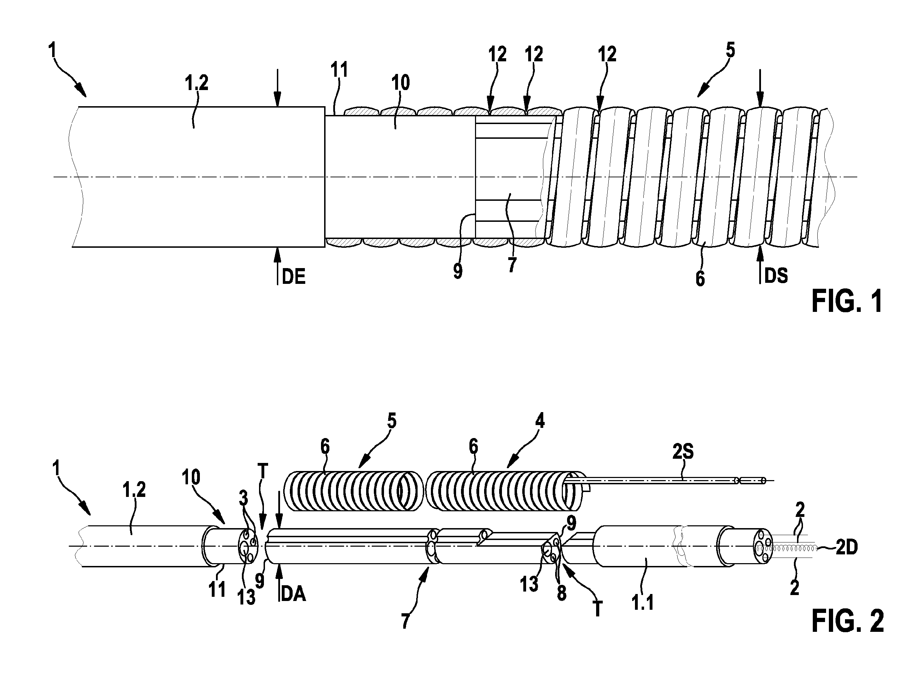

Wie aus den Fig. 1 und 2 deutlich wird, weist die ausschnittsweise gezeigte Elektrodenvorrichtung zur kardiovaskulären Anwendung einen langgestreckten Elektrodenkörper 1 auf, der an einer Trennstelle T unterbrochen und somit in zwei Teilstücke 1.1, 1.2 aufgeteilt ist. Der Elektrodenkörper 1 ist ein langgestrecktes, schlauchartiges Gebilde aus einem isolierenden Material, in dem mehrere Zuleitungen 2 zur entsprechenden Elektroden zur Erfassung kardiologischer Signale und/oder zur Abgabe elektrokardiologischer Reizsignale in Lumen 3 geführt sind. In den Fig. 1 und 2 sind von diesen Elektroden zwei Schockwendeln 4, 5 gezeigt, die aus einem eng gewickelten Banddraht 6 gebildet sind. Der Außendurchmesser DS der Schockwendeln 4, 5 entspricht dabei im Wesentlichen dem Außendurchmesser DE des Elektrodenkörpers 1 in dessen beiden Abschnitten 1.1, 1.2. In Fig. 2 ist im Übrigen die Zuleitung 2S der Schockwendel 4 gezeigt.

Die weiteren, lediglich in Fig. 1 angedeuteten Zuleitungen 2 sind als nicht-dehnbare Seile ausgebildet.

In die Trennstelle T zwischen den beiden Abschnitten 1.1, 1.2 des Elektrodenkörpers 1 ist ein Ausgleichs-Schlauchabschnitt 7 eingefügt, der Aufnahmen 8 in Form von den Lumen 3 entsprechenden Lumen aufweist. Wie in Fig. 1 und 2 nicht explizit dargestellt ist, sind diese Aufnahmen 8 entlang der Längsrichtung des Ausgleichs-Schlauchabschnittes 7 schraubenlinienförmig ausgebildet, so dass die darin geführten Zuleitungen 2 ebenfalls schraubenlinienförmig gewendelt darin verlaufen. Der Außendurchmesser DA des Ausgleichs-Schlauchabschnittes 7 st dabei an den Innendurchmesser der Schockwendel 4, 5 angepasst, so dass der Ausgleichs-Schlauchabschnitt 7 innerhalb der Schockwendel 4, 5 angeordnet ist. Die quer verlaufenden Fügeseiten 9 des Schlauchabschnittes 7 sind dabei gegenüber den äußeren Enden 10 der Schockwendeln 4, 5 nach innen zurückgezogen. Zur Verbindung mit den Abschnitten 1.1, 1.2 des Elektrodenkörpers 1 weisen diese im Durchmesser reduzierte Absätze 11 auf, deren Außendurchmesser dem Durchmesser DA der Ausgleichs-Schlauchabschnitte 7 entspricht. Damit greifen die Absätze 11 in die Schockwendeln 4,5 bis zur Fügeseite 9 des Schlauchabschnittes 7 ein. Letzterer ist mit den Abschnitten 1.1, 1.2 des Elektrodenkörpers 1 dann jeweils lösungsdicht hermetisch verklebt. Ferner sind die Zwischenräume 12 zwischen den Schockwendeln 4,5 und dem Ausgleichs-Schlauchabschnitt 7 durch ein nicht näher dargestelltes Vergussmittel beispielsweise in Form von flüssigem Silikon-Kautschuk oder Silikon-Klebstoff verfüllt. Da der Ausgleichs-Schlauchabschnitt 7 selbst aus Silikongummi extrudiert oder mittels Spritzgießen aus flüssigem Silikon-Kautschuk hergestellt ist, verbinden sich die Silikon-Materialien des Schlauchabschnittes 7 und des Vergussmittels unlösbar.

In Fig. 2 ist im Übrigen noch ein zentrales, gradliniges durchgehendes Lumen 13 für eine wendelförmige, dehnbare Zuleitung 2D einer nicht näher dargestellten Spitzenelektrode gezeigt. Die Zuleitungen 2, 2S und 2D sind der Übersichtlichkeit halber nur rechts vor dem Abschnitt 1.2 des Elektrodenkörpers 1 einlaufend dargestellt.

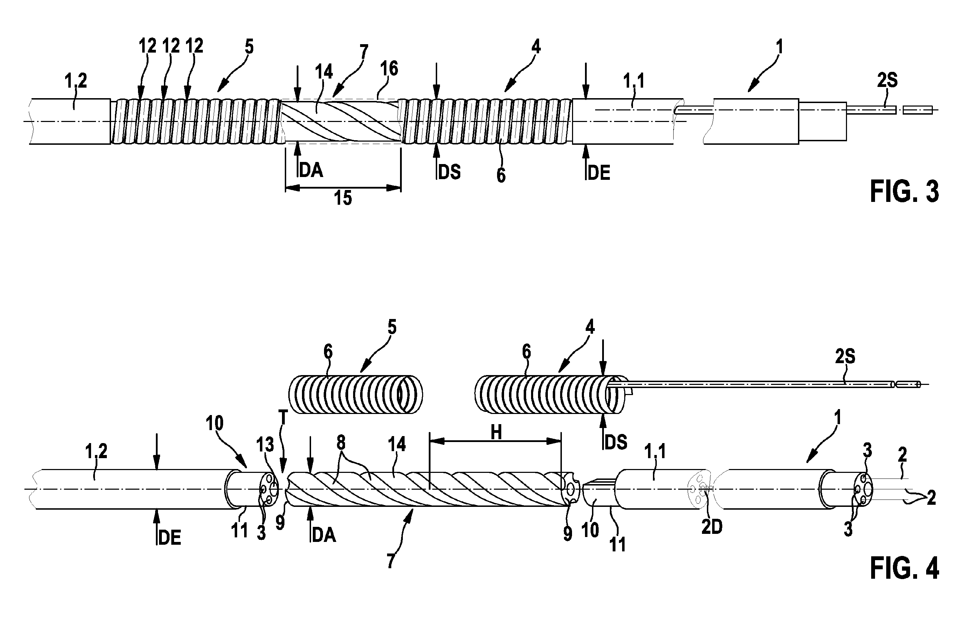

In den Fig. 3 und 4 ist wiederum der Ausschnitt eines Elektrodenkörpers 1 gezeigt, bei dem zwischen zwei Abschnitten 1.1 und 1.2 ein Ausgleichs-Schlauchabschnitt 7 eingesetzt ist. Letzterer weist bei dieser Ausführungsform Aufnahmen 8 auf, die als schraubenlinienförmige Nuten auf der Außenseite 14 ausgebildet sind. Letztere korrespondieren mit den drei engen Lumen 3 im Elektrodenkörper 1 und übernehmen die dort verlaufenden Zuleitungen 2, die wiederum nur rechts in Fig. 4 angedeutet sind. Ein zentrales Lumen 13 nimmt eine wendelförmige, dehnbare Zuleitung 2D auf. Der Ausgleichs-Schlauchabschnitt 7 ist mit seinen Fügeseiten 9 analog der Ausführungsform gemäß Fig. 1 und 2 unter den beiden Schockwendeln 4 und 5 angeordnet und gleichermaßen hermetisch dicht mit den Abschnitten 1.1, 1.2 des Elektrodenkörpers 1 verbunden. Der Eingriff dieser Abschnitte 1.1, 1.2 in die Schockwendeln 4, 5 ist wiederum über Absätze 11 gewährleistet.

Zwischen den beiden Schockwendeln 4, 5 verbleibt ein Zwischenraum 15, in dem eine gestrichelt dargestellte Abdeckhülse 16 auf den Ausgleichs-Schlauchabschnitt 7 mit den nutenartigen Aufnahmen 8 aufgesetzt und hermetisch dicht damit verklebt ist. Der Außendurchmesser DA dieser Abdeckhülse 16 entspricht dem Außendurchmesser DS der Schockwendeln 4, 5, so dass sich insgesamt ein isodiametrischer Aufbau der Elektrodenvorrichtung gemäß Fig. 3 und 4 ergibt. Dies trifft auch für die in Fig. 1 und 2 gezeigte Ausführungsform zu.

Entsprechend den drei Lumen 3 im Elektrodenkörper 1 weist der Ausgleichs-Schlauchabschnitt 7 im Übrigen drei nutenförmige Aufnahmen 8 auf, die jeweils eine Ganghöhe H von etwa dem 4-fachen des Außendurchmesser DA des Ausgleichs-Schlauchabschnittes 7 in Fig. 3 und 4 entspricht.

## Patentansprüche

1. Elektrodenvorrichtung, insbesondere zur kardiovaskulären Anwendung, umfassend
- einen lang gestreckten Elektrodenkörper (1) aus einem isolierenden Material,
- mehrere Elektroden (4, 5) zur Erfassung elektrokardiologischer Signale und/oder zur Abgabe elektrokardiologischer Reizsignale, und
- für die elektrische Anbindung der Elektroden (4, 5) dienende Zuleitungen (2, 2S, 2D), insbesondere nicht-dehnbare Seile oder Litzen, welche Zuleitungen (2, 2S 2D) jeweils im Elektrodenkörper (1) vorzugsweise in zugeordneten Lumen (3, 13) geführt sind,
**gekennzeichnet durch**
- einen in eine Trennstelle (T) im Elektrodenkörper (1) eingefügten Ausgleichs-Schlauchabschnitt (7) mit maximal dem Elektrodenkörper (1) entsprechenden Außendurchmesser (DA), wobei in den Ausgleichs-Schlauchabschnitt (7) schraubenlinienförmige Aufnahmen (8) für jeweils eine Zuleitung (2) eingebracht sind und der Ausgleichs-Schlauchabschnitt (7) an seinen Fügeseiten (9) zum Elektrodenkörper (1) hermetisch dicht mit diesem verbunden ist.

2. Elektrodenvorrichtung nach Anspruch 1, **dadurch gekennzeichnet, dass** die Aufnahmen (8) als schraubenlinienförmige Lumen ausgebildet sind.

3. Elektrodenvorrichtung nach Anspruch 1, **dadurch gekennzeichnet, dass** die Aufnahmen (8) als schraubenlinienförmige Nuten auf der Außenseite (14) des Ausgleichs-Schlauchabschnitts (7) ausgebildet sind.

4. Elektrodenvorrichtung nach Anspruch 3, **dadurch gekennzeichnet, dass** der Ausgleichs-Schlauchabschnitt (7) mit seinen die Zuleitungen (2) aufnehmenden nutenförmigen Aufnahmen (8) außenseitig mit einer Abdeckhülse (16) umschlossen ist.

5. Elektrodenvorrichtung nach Anspruch 1, umfassend ferner mindestens eine Schockwendel (4, 5) auf dem Elektrodenkörper (1), die aus einem leitfähigen, schraubenlinienförmig gewickelten Wendeldraht (6) gebildet ist, **dadurch gekennzeichnet, dass** der Ausgleichs-Schlauchabschnitt (7) zumindest teilweise unter der Schockwendel (4, 5) angeordnet ist, wobei mindestens eine Fügeseite (9) des Ausgleichs-Schlauchabschnitts (7) zum Elektrodenkörper (1) unter der Schockwendel (4, 5) liegt und der Elektrodenkörper (1) mit einem im Durchmesser reduzierten Absatz (11) in die Schockwendel (4, 5) bis zur Fügeseite (9) des Ausgleichs-Schlauchabschnitts (7) eingreift.

6. Elektrodenvorrichtung nach Anspruch 5, **dadurch gekennzeichnet, dass** die Zwischenräume (12) zwischen dem Ausgleichs-Schlauchabschnitt (7) und der Schockwendel (4, 5) mit einem Vergussmittel verfüllt werden.

7. Elektrodenvorrichtung nach einem der vorgenannten Ansprüche, umfassend ferner eine zentral geführte, dehnbare Zuleitung (2D), insbesondere Wendelleitung, **dadurch gekennzeichnet, dass** der Ausgleichs-Schlauchabschnitt (7) ein vorzugsweise zentrales, geradlinig durchgehendes Lumen (13) für die dehnbare Zuleitung (2D) aufweist.

8. Elektrodenvorrichtung nach einem der vorgenannten Ansprüche, **dadurch gekennzeichnet, dass** der Ausgleichs-Schlauchabschnitt (7) aus Silikongummi besteht.

9. Elektrodenvorrichtung nach Anspruch 8, **dadurch gekennzeichnet, dass** der Ausgleichs-Schlauchabschnitt (7) extrudiert ist.

10. Elektrodenvorrichtung nach Anspruch 8, **dadurch gekennzeichnet, dass** der Ausgleichs-Schlauchabschnitt (7) aus Flüssig-Silikongummi spritzgegossen ist.

11. Elektrodenvorrichtung nach Anspruch 4, **dadurch gekennzeichnet, dass** der Ausgleichs-Schlauchabschnitt (7) aus Silikongummi oder Silikon-Polyurethan-Co-Polymer und die Abdeckhülse (16) aus Silikon-Polyurethan-Co-Polymer oder Polyurethan bestehen.

12. Elektrodenvorrichtung nach einem der vorgenannten Ansprüche, **dadurch gekennzeichnet, dass** die Ganghöhe (H) einer schraubenlinienförmigen Aufnahme (8) dem 3-fachen bis 5-fachen Außendurchmesser (DA) des Ausgleichs-Schlauchabschnittes (7) entspricht.
